Europäisches Patentamt

European Patent Office

Office européen des brevets

(11) Publication number: **0 261 284**
**A1**

(12) **EUROPEAN PATENT APPLICATION**

(21) Application number: 86307289.8

(22) Date of filing: 23.09.86

(51) Int. Cl.4: **A61B 17/02**

(43) Date of publication of application:
**30.03.88 Bulletin 88/13**

(84) Designated Contracting States:
**AT BE CH DE FR GB IT LI NL SE**

(71) Applicant: **Wigoda, Luis T.**
**345 North Shore Drive**
**Miami Beach Florida 33141(US)**

(72) Inventor: **Wigoda, Luis T.**
**345 North Shore Drive**
**Miami Beach Florida 33141(US)**

(74) Representative: **Miller, Joseph et al**
**J. MILLER & CO. Lincoln House 296-302 High**
**Holborn**
**London WC1V 7JH(GB)**

(54) **Retractor apparatus.**

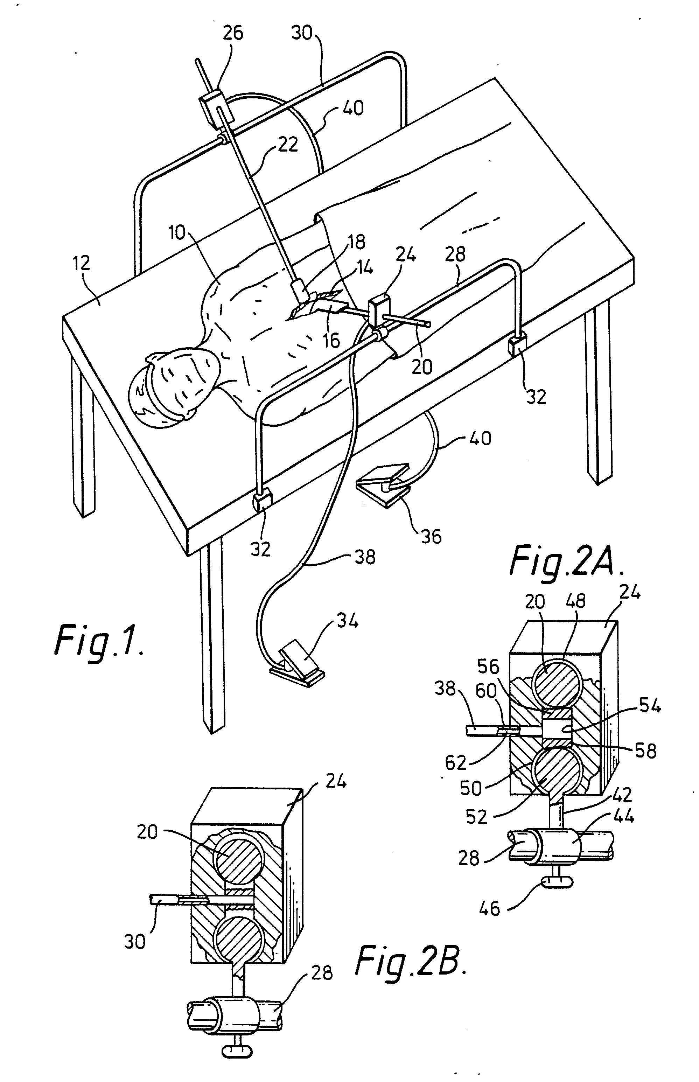

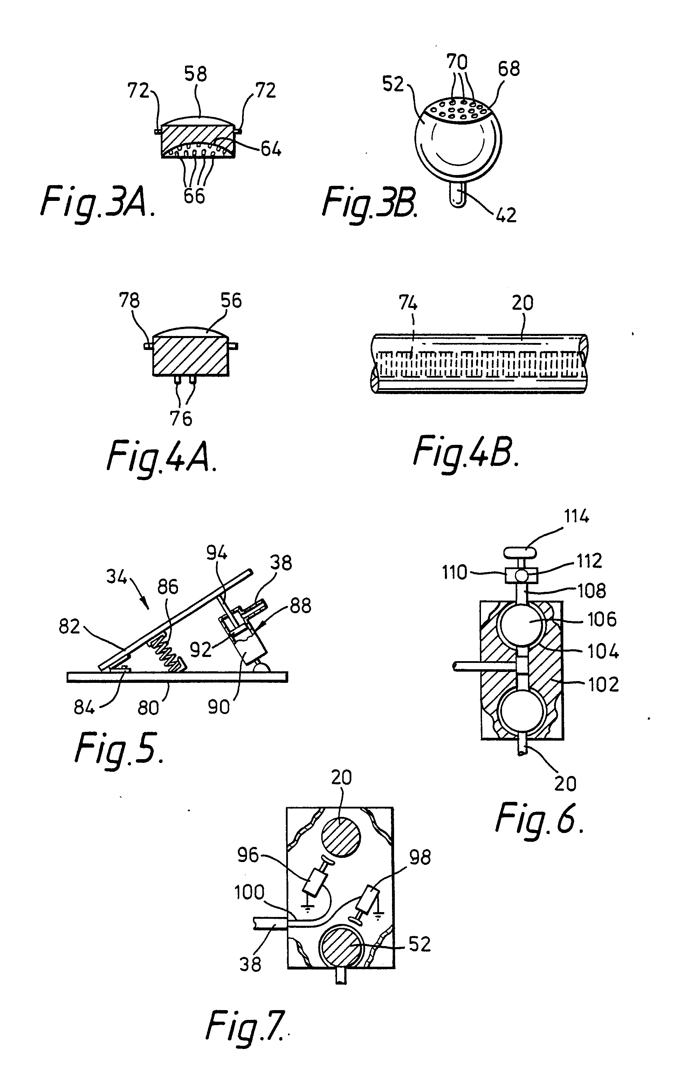

(57) Retractor apparatus for use during a surgical procedure for holding back tissue (14) of a patient (10) undergoing surgery, said apparatus having at least one retractor (16,20 or 18,22) adapted to hold back said tissue (14), joint means (24 or 26) for holding said retractor (16,20 or 18,22) in a selected position, and attachment means (28 or 30) for holding said joint means (24 or 26), said attachment means (28 or 30) being adapted for attachment relative to an operating platform (12) supporting the patient (10), said joint means (24 or 26) being controlled to release or lock said retractor (16,20 or 18,22) by actuated controller means (34 or 36), said joint means (24 or 26) being characterised by controllable lock means (Figures 2A and 2B or Figure 7) for releasing said retractor (16,20 or 18, 22) for positioning when said controller means (34 or 36) is actuated and for locking said retractor (16,20 or 18,22) when said controller means (34 or 36) is de-actuated, said retractor (16,20 or 18,22) being positionable when said lock means (Figure 2A and 2B or Figure 7) is de-actuated by linear movement through said joint means (24 or 26) in a direction away from said attachment means (28 or 30) and by rotational movement of said joint means (24 or 26) in a direction about said attachment means (28 or 30).

Fig.1.

Fig.2A.

## "RETRACTOR APPARATUS"

This invention relates to retractor apparatus for use during a surgical procedure and, although the invention is not so restricted, it relates more particularly to such apparatus which can be locked or released in response to the actuation of a foot pedal.

For many years retractors have been used during a surgical procedure. Typically at least two retractors are used to pull away different layers of tissue around an incision or body opening to allow the surgeon to work in a desired exposed area. In many instances, two doctors are required, one to hold the retractors and the other to do the actual surgical procedure. The doctor holding the retractors may become tired very quickly because, in some instances, significant pressure must be applied to the retractor to maintain the desired area clear for the surgeon to proceed.

In surgical applications, mechanical devices have been suggested in the past to replace the second physician holding the retractors. An example of such a mechanical device is described in US-A-4,355,631 in the name of Bruce A. LeVahn. Other types of devices include those sold by Thompson Surgical Instruments of Barrington, Illinois. Typical of these sets of mechanically held retractors is a bar which runs along the side of the operating table. The retractors are connected to rods which can be affixed to mechanisms attached to the bar. The mechanism allows the retractor to be rotated both about and parallel to the bar as well as moved laterally along the bar and towards and away from the patient. Thumb screws or other similar apparatus must be manually loosened to allow the retractor rod to be moved and thereafter tightened when the rod is properly positioned or repositioned. The problem with these types of mechanically held retractors is that, during many surgical procedures, it is necessary to readjust the retractors. Sometimes this movement is permanent and at other times it will be of a more temporary nature to allow the surgeon to reach a particular area. Thereafter the retractor would desirably be placed back in the original position. Because of the manual intervention requirement by the surgeon to loosen the screws on the connectors, the surgeon's concentration may be distracted since he physically must use both hands to loosen the thumb screw and reposition the retractor devices. This means that the surgery itself must stop while the retractors are repositioned. Stopping the surgery is particularly burdensome on the surgeon when it is desired to move retractors for only a short time to reach a specific area within the incision.

It would be more advantageous for the surgeon performing the surgery to have retractors which can be automatically loosened and manually moved without requiring the intervention of both of the surgeon's hands. If, for instance, the surgeon were allowed to depress a foot pedal to momentarily free the retractors for movement, he could use one hand to reposition a retractor while still using the other hand to perform the necessary surgical procedure. Thereafter, he could reposition the retractors in the desired position and release the footpedal, thereby locking the retractors.

According, therefore, to the present invention, there is provided a retractor apparatus for use during a surgical procedure for holding back tissue of a patient undergoing surgery, said apparatus having at least one retractor adapted to hold back said tissue, joint means for holding said retractor in a selected position, and attachment means for holding said joint means, said attachment means being adapted for attachment relative to an operating platform supporting the patient, said joint means being controlled to release or lock said retractor by actuated controller means, said joint means being characterized by controllable lock means for releasing said retractor for positioning when said controller means is actuated and for locking said retractor when said controller means is de-actuated, said retractor being positionable when said lock means is de-actuated by linear movement through said joint means in a direction away from said attachment means and by rotational movement of said joint means in a direction about said attachment means.

Preferably said controllable lock means includes hydraulic means.

The said controller means may include foot actuated means.

The said retractor is preferably connected at the end of a rod which is slidable through and may be locked in said joint means, said joint means being coupled to said attachment means by a lockedly rotatable ball joint connection. Thus the said rod and said ball joint connection may be locked in response to a hydraulically actuated piston positioned thereagainst.

The said controller means include a foot actuated hydraulic piston and cylinder assembly for drawing fluid away from said joint means upon actuation thereof.

The said controller means may include spring means to reset said controller means after actuation.

An hydraulic cylinder may be positioned between said rod and said ball joint connection, a pair of pistons being positioned within said cylinder, said controller means being hydraulically coupled to said cylinder at a point between said pair of pistons.

Said rod and ball joint connections may be locked in response to an electrically actuated solenoid piston positioned thereagainst.

One preferred embodiment of the present invention is hereafter described, merely by way of example, with specific reference being made to the following figures, in which:-

Figure 1 shows a retractor apparatus according to the present invention;

Figures 2A and 2B show a locking mechanism which forms part of the apapratus of Figure 1 and which comprises a hydraulic release and lock;

Figures 3A and 3B show a portion of the apparatus of Figure 1 comprising a lockable ball joint;

Figures 4A and 4B illustrate another portion of the apparatus of Figure 1 showing a locking mechanism for a rod attached to a retractor of the apparatus;

Figure 5 shows a foot pedal forming part of the apparatus of Figure 1;

Figure 6 shows an alternative embodiment of a locking mechanism; and

Figure 7 shows another alternative embodiment of a locking mechanism.

Referring now to Figure 1, a patient 10 is shown positioned on an operating table 12. The patient 10 has an incision 14 in his body, the incision typically being made by a surgeon during a surgical procedure. In order to maintain the tissue beneath the incision 14 in a separated condition so that the surgeon is able to operate within the body at the desired location, a pair of retractors 16 and 18 at the end of retractor rods 20 and 22 are used. The retractor rods 20 and 22 are held by retractor joints 24 and 26 respectively. Each of the joints 24 and 26 are in turn held on rods 28 and 30 which are affixed to the side of operating table 12 by members 32 in a conventional manner. Joints 24 and 26 may alternatively be coupled to mechanisms remote from table 12, such as floor stands or ceiling extensions.

The retractor rods 20 and 22 may slide through joints 24 and 26 to be positioned lengthwise at the appropriate position of incision 14. Joints 24 and 26 slide along rods 28 and 30 respectively until retractors 16 and 18 are properly positioned. Joints 24 and 26 are also tiltable to position retractors 16 and 18 at the proper angle. Thus, by sliding the joints 24 and 26 to their proper position along rods 28 and 30 and by tilting the joints 24 and 26 and extending the retractor rods 20 and 22 to the proper position, the tissue beneath the incision 14 may be maintained separated in a manner to allow the surgery to be performed. To be used in surgical application, each of the components heretofore described must be of a material capable of being sterilized, such as stainless steel.

Each of the joints 24 and 26 are respectively coupled to a foot controller 34 and 36 by a controller cable 38 and 40. Foot controller 34 and 36 may control the locking of joint 24 and 26 either in a hydraulic, electro-mechanical or mechanical manner, in which case the cable 38 would be a corresponding hydraulic line, electrical wire or mechanical cable. For the purposes of Figure 1, whenever one of the foot controllers 34 or 36 is depressed, a locking mechanism within the joint 24 or 26 corresponding to that controller is released so that the retractor rod 20 can be moved inward or outward from the joint 24 or 26. In addition, the joint 24 or 26 is able to be tilted or rotated to position the retractor properly within the incision 14. Once initially positioned, small movements of the retractor 16 or the retractor 18 may be accomplished by the surgeon during the middle of a surgical procedure by the surgeon merely depressing one of the foot controllers 34 or 36 and moving the retractor with his free hand.

In Figure 1, the incision 14 is shown in generally the chest portion of the patient 10. It should be understood that the incision 14 could be anywhere on the patient 10 and that retractors 16 and 18 could be used where no incision is required, such as during gynaecological surgery.

Referring now to Figures 2A and 2B, a hydraulic version of the joint 24 is hereafter described. It should be understood that the joint 26 would be identical to the joint 24 described in Figures 2A and 2B. The joint 24 is coupled to the rod 28 by a support rod 42 coupled to a slide ring 44 which in turn is secured to the rod 28 by a thumb screw 46. The support ring 44 may be moved along the rod 28 and to the desired position generally perpendicular to that part of the incision 14 where the retractor 16 is to be positioned. By selecting the rotary position where the thumb screw 46 is secured, the joint 24 may be tilted towards the incision 14 as desired.

The joint 24 is a generally solid member having a cylindrical opening 48 therethrough through which the retractor rod 20 is inserted. A spherical opening 50 is also provided into which a generally spherical ball 52 is positioned. The bottom of the spherical ball 52 is connected to the support rod 42. Both openings 48 and 50 are slightly larger than the retractor rod 20 and the ball 52 so that in the absence of a locking action, to be described hereafter, each may slide or rotate within the opening. A hydraulic passage 54 is positioned between the cylindrical opening 48 and the spherical opening 50. Within the hydraulic passage 54 are positioned a pair of hydraulic pistons 56 and 58 associated with the respective openings 48 and 50. A second hydraulic passage 60 is further provided from hydraulic passage 54 to a hydraulic fitting 62 to which the controller cable 38 is attached. In the hydraulic version shown in Figures 2A and 2B, the controller cable 38 may be a hydraulic hose.

As long as fluid pressure is maintained within the cable 38 and into the passage 54 through the passage 60, the two pistons 56 and 58 are maintained against the retractor rod 20 and the ball 52. The manner in which the pistons 56 and 58 maintain the retractor rod 20 immobile is described in more detail in Figures 3A and 3B, and 4A and 4B.

Referring to Figure 2B, as fluid is moved out of the passage 54 through the passage 60 and the controller cable 38, the pistons 56 and 58 move away from the rod 20 and the ball 52 to the positions shown in Figure 2B. In this instance, the retractor rod 20 may be moved through the opening 48 towards or away from the incision 14. Similarly, the joint 24 may be pivoted about the ball 52 so that its angular position may be modified as desired. After the movements of either the rod 20 or the joint 24 about the ball 52 have been accomplished, the hydraulic fluid is forced back into the passages 60 and 54, and the pistons 56 and 58 return to the locking position shown in Figure 2A.

Referring now to Figures 3A and 3B, the details of piston 58 and ball 52 are shown. The piston 58, shown in Figure 3A, is cut through the centre thereof to show the curvature of the contact face 64. The ·contact face 64 is generally concave-spherical in shape, having approximately the same radius as the radius of the ball 52. The outer shape of the piston 58 is generally cylindrical in shape having a radius of slightly less than the radius of the hydraulic passage 54. Extending down from the radial contact face 64 of the piston 58 are a plurality of small extensions or pimples 66. While only two rows of pimples 66 are shown in Figure 3A, it should be understood that the entire contact face surface includes similar pimples 66. In addition, an "O"-ring 72 is positioned about the piston 58 to keep the hyraulic fluid from flowing into contact with the ball 52 and out of the bottom of the joint 24.

In Figure 3B, that ball 52 is shown generally as a sphere connected to the support rod 42. A contact surface 68 of the ball 52 is positioned opposite to the rod 42 and includes a plurality of pimples 70 extending upwardly therefrom. The pimples 66 and 70 are arranged to mesh within one another so that, when the piston 58 is moved by hydraulic pressure against the contact surface 68 of the ball 52, no relative movement between the piston 58 and the ball 52 can occur. This in turn maintains the joint 24 in a fixed position relative to the various movements it can make around the ball 52.

Referring now to Figures 4A and 4B, the piston 56 and the retractor rod 20 are respectively shown therein. The retractor rod 20, shown in Figure 4B, includes a locking surface 74 having a series of slot openings. The piston 56, shown in Figure 4A, includes a plurality of, for instance two, finger extensions extending from the bottom thereof, the finger extensions being sized to fit within a corresponding plurality of the slots on the locking surface 74. When so engaged, the retractor rod 20 is held firmly in a fixed position. The piston 56 also includes an "O"-ring 78 to prevent the hydraulic fluid from getting into the opening 48.

Referring now to Figure 5, a more detailed view of foot controller 34 is shown. The controller 34 consists of a base 80 and a foot pedal 82 connected to the base 80 by a hinge 84. The foot pedal 82 is maintained normally in the position shown by a spring 86. Also coupled between the base 80 and the foot pedal 82 is a piston and cylinder assembly 88 which consists of a cylinder 90, a piston 92 and a piston rod 94. The hydraulic hose controller cable 38 is coupled to the upper end of the cylinder 90 so as to be on the rod side of the piston 92. Thus, when the foot pedal 82 is depressed, the piston rod 94 drives the piston 92 deeper into the cylinder fluid from the cable 38 and out from the hydraulic passage 54 and the opening 60 in Figure 2A. This, in turn, releases the pistons 56 and 58 from engaging the retractor rod 20 and the ball 52 so that they can be readily moved as previously explained. When the foot pedal 82 is released, the spring 86 forces the foot pedal 82 back to the position shown in Figure 5. This, in turn, raises the piston 92, thus forcing fluid back into the controller cable 38 and into the hydraulic passage 54, which moves the pistons 56 and 58 to the position shown in Figure 2A, thus locking the retractor rod 20 and the ball 52. The amount of fluid in the cable 38, the passages 54 and 60 and the upper portion of the cylinder 90 will determine how far the pedal 82 is moved after release. The spring 86 causes a pressure to be applied by the piston 92 against this fluid, which pressure is applied throughout the fluid to lock the pistons 56 and 58 against the rod 20 and the ball 52.

Referring now to Figure 6, an alternative embodiment of a joint 102 is shown. The joint 102 differs from the joint 24 in that the cylindrical opening 48 holding the rod 20 is replaced by a spherical opening 104 holding a ball 106 similar to the ball 52. A stem rod 108 from the ball 106 is coupled to a holder 110 for holding a retractor 112 which may be tightened by a thumb screw 114. The joint 102 has the advantage that the retractor 112 may be moved with more degrees of freedom than was the case with the joint 24.

Referring now to Figure 7 another alternative embodiment of a foot controlled retractor system is shown in which the rod 20 and the ball 52 are locked through the use of electric solenoids 96 and 98. The end of the electric solenoids 96 and 98 are similar to the pistons 56 and 58 shown in Figures 3A and 4A and the rod 20 and ball 52 are similar to that shown in Figures 3B and 4B. Wires 100 extending through the controller cable 38 are used to energize the solenoids 96 and 98. In Figure 5, the foot pedal 34 would be modified so that when the pedal 82 is depressed, short circuit connections energizing the wires 100 would be made.

Other types such as mechanical linkages could be utilized to engage or free the rod 20 and ball 52. For example, rather than utilizing the rod 28 attached to the operating table 12, as shown in Figure 1, the joint 24 or 102 could be attached to one or more extension members or arms from either a fixed floor stand or the ceiling above table 12. The arms could be interlocked by joints to be positioned so that the retractors 16 are properly positioned when inserted in the joint 24 or 102, at the end of the arms. Such interlocked arms could also be attached to the table 12.

## Claims

1. Retractor apparatus for use during a surgical procedure for holding back tissue (14) of a patient (10) undergoing surgery, said apparatus having at least one retractor (16,20 or 18,22) adapted to hold back said tissue (14), joint means (24 or 26) for holding said retractor (16,20 or 18,22) in a selected position, and attachment means (28 or 30) for holding said joint means (24 or 26), said attachment means (28 or 30) being adapted for attachment relative to an operating platform (12) supporting the patient (10), said joint means (24 or 26) being controlled to release or lock said retractor (16,20 or 18,22) by actuated controller means (34 or 36), said joint means (24 or 26) being characterised by controllable lock means (Figures 2A and 2B or Figure 7) for releasing said retractor (16,20 or 18, 22) for positioning when said controller means (34 or 36) is actuated and for locking said retractor (16,20 or 18,22) when said controller means (34 or 36) is de-actuated, said retractor (16,20 or 18,22) being positionable when said lock means (Figure 2A and 2B or Figure 7) is de-actuated by linear movement through said joint means (24 or 26) in a direction away from said attachment means (28 or 30) and by rotational movement of said joint means (24 or 26) in a direction about said attachment means (28 or 30).

2. Apparatus according to claim 1 characterised in that said controllable lock means (Figures 2A and 2B or Figure 7) includes hydraulic means (Figures 2A and 2B).

3. Apparatus according to claim 1 or 2 characterised in that said controller means (34 or 36) includes foot actuated means (34 or 36).

4. Apparatus according to any preceding claim characterised in that said retractor (16 or 18) is connected at the end of a rod (20 or 22) which is slidable through and may be locked in said joint means (24 or 26), said joint means (24 or 26) being coupled to said attachment means (28 or 30) by a lockedly rotatable ball joint (52) connection.

5. Apparatus according to claim 4 characterised in that said rod (20 or 22) and said ball joint (52) connection are locked in response to a hydraulically actuated piston (58) positioned thereagainst.

6. Apparatus according to claim 5 characterised in that said controller means (34 or 36) includes a foot actuated hydraulic piston (92,94) and cylinder (90) assembly (88) for drawing fluid away from said joint means (24 or 26) upon actuation thereof.

7. Apparatus according to claim 6 characterised in that said controller means (34 or 36) includes spring means (86) to reset said controller means (34 or 36) after actuation.

8. Apparatus according to claim 4 or 5 characterised in that an hydraulic cylinder (54) is positioned between said rod (20 or 22) and said ball joint (52) connection, a pair of pistons (56,58) being positioned within said cylinder (54), said controller means (34 or 36) being hydraulically coupled to said cylinder (54) at a point between said pair of pistons (56 and 58).

9. Apparatus according to claim 4 characterised in that said rod (20 or 22) and ball joint (52) connections are locked in response to an electrically actuated solenoid piston (96) positioned thereagainst.

10. Retractor apparatus for use during a surgical procedure for holding back tissue of a patient undergoing surgery, said apparatus comprising: at least one retractor adapted to hold back said tissue; attachment means adapted for attachment relative to said operating platform supporting said patient; joint means for affixing said retractor to said attachment means in the position to hold back said tissue, said joint means including controllable lock means for releasing said retractor for positioning and thereafter locking said positioned retractor; and actuated controller means coupled to said lock means for controlling said lock means to release said retractor upon actuation thereof and for controlling said lock means to lock said retractor upon deactuation thereof.

*Fig.1.*

*Fig.2A.*

*Fig.2B.*

Fig.3A.

Fig.3B.

Fig.4A.

Fig.4B.

Fig.5.

Fig.6.

Fig.7.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | US-A-4 355 631 (LeVAHN) <br> * Column 2, line 64 - column 3, line 20; figure 1 * | 1-10 | A 61 B 17/02 |
| Y | US-A-3 858 578 (MILO) <br> * Column 4, lines 30-38; figures * | 1-10 | |
| A | US-A-3 638 973 (POLETTI) <br> * Column 5, lines 45-75; figures * | 1-10 | |
| A | US-A-3 158 071 (GUT) | | |
| A | US-A-3 915 436 (MATSON) | | **TECHNICAL FIELDS SEARCHED (Int. Cl.4)** <br><br> A 61 B |

The present search report has been drawn up for all claims

| Place of search | Date of completion of the search | Examiner |
|---|---|---|
| THE HAGUE | 01-06-1987 | GLAS J. |